# EUROPEAN PATENT SPECIFICATION

(11) **EP 2 243 445 B1**
(45) Date of publication and mention of the grant of the patent: **21.12.2011**
(21) Application number: 10170058.1
(22) Date of filing: 19.02.2007
(51) Int. Cl.: A61F 2/40, A61F 2/46

(54) **A glenoid component of a shoulder joint prosthesis**
Gelenkpfannenteil einer Schultergelenksprothese
Composant glenoide d'une prothèse d'articulation de l'épaule

(30) Priority: 23.02.2006 GB 0603572
(43) Date of publication of application: 27.10.2010
(62) Divisional of application: 07705623.2
(73) Proprietor: DePuy (Ireland), Co Cork (IE)
(72) Inventor: Dewilde, Lieven, 9000, Gent (BE); Karelse, Anne, 9190, Stekene (BE)
(74) Representative: Belcher, Simon James

(56) References cited:
- EP-A1- 1 426 023
- WO-A2-02/067811
- FR-A1- 2 843 293

## Description

This invention relates to a glenoid component of a shoulder joint prosthesis and to a method of planning a shoulder joint replacement surgical procedure.

A glenoid component of a shoulder joint prosthesis is used in the repair and replacement of a diseased or damaged shoulder joint. The glenoid component is fastened in the glenoid region to the scapula and provides a surface with which a humeral implant contacts and articulates.

One type of common glenoid implant is a "metal backed" glenoid implant. A metal backed glenoid implant comprises a frame part for attachment to the glenoid region of the scapula, and a body part which provides the surface which the humeral implant contacts and articulates. As will be understood, if the metal backed glenoid implant is part of an anatomic shoulder prosthesis, then the body part will be a "cup" which presents a rounded concave surface. However, if the metal backed glenoid implant is part of a reverse shoulder prosthesis, then the body part will be a "head" which presents a rounded convex surface.

It is important that the frame part can be secured to the scapula sufficiently strongly to withstand forces exerted on the glenoid implant. During implantation, the location of the frame part relative to the glenoid is generally selected so that the joint prosthesis matches as closely as possible the anatomical configuration of the natural joint. Accordingly, it can be desirable to prevent any movement of the frame relative to the glenoid once implanted. Also, an improperly anchored frame can result in additional damage being caused to the scapula. As disclosed in WO-A-02/067811, a glenoid component can be fastened to a patient's glenoid by means of bone screws which extend through holes in the component.

The invention provides a method of planning a shoulder joint replacement surgical procedure, as defined in claim 1.

Preferably, the screws are arranged so that the distance measured between their respective axes decreases with increasing distance away from the medial surface of the frame towards the scapula.

The present invention can use a frame part of a glenoid component which provides at least two bores, each which are configured so that the distance measured between bone screws received therethrough decreases with increasing distance away from the frame.

Preferably, the method includes the step of locating a portion of the surface of the scapula which is generally circular and generally planar, and mounting the frame part of the glenoid component on that portion of the scapula surface.

Preferably, the method of the invention makes use of a glenoid component as discussed below.

The glenoid component of a shoulder joint prosthesis which is intended for fixation to the glenoid region of a scapula, comprises a body part of the prosthesis which presents a surface for articulation with the corresponding articulating surface of a humeral component, and a frame part which has: a lateral surface on which the body part can be mounted; a medial surface generally opposite the lateral surface; and first and second bores that extend between the lateral and medial surfaces for receiving screws so as to fasten the frame part to the scapula; in which the bores are configured so that their axes converge, so that the distance measured between the axes decreases with increasing distance away from the medial surface of the frame towards the scapula.

It has been found that a frame fastened to the glenoid region of a scapula by bone screws that converge toward each other away from the medial surface of the frame ("converging screws"), can improve the anchorage of the frame on the scapula. The use of converging screws can increase the chance of the screws becoming anchored within structurally sound portions of the scapula. In particular, the screws can be directed into the lateral border pillar and the spine pillar of the scapula. The problem of bone screws being improperly and/or insufficiently anchored is particularly an issue during revision of a glenoid implant. Each implantation requires the creation of holes in the scapula, for example to receive bone screws and/or pegs. Accordingly, achieving adequate anchorage of a frame during revision of a glenoid implant can be difficult due to the deterioration in the structural integrity of the bone, caused by the presence of a number of holes. It has been found that the anchorage of frames of revised glenoid implants can be improved by using converging screws.

The frame of the glenoid component has the advantage that screws received through its bores are guided by the bores so that the screws converge toward each other as you travel away from the medial surface of the frame. Accordingly, the frame provides the advantages associated with anchoring a frame to a scapula using converging screws discussed above.

The cross-sectional shape of the frame can be any regular or irregular shape. For example, the cross-sectional shape of the frame can be rectangular, or hexagonal. Preferably, the cross-sectional shape of the frame is generally rounded, for example oval or generally elliptical, especially generally circular. A rounded frame can best match the anatomical shape of the glenoid region.

Preferably, the frame has a substantially planar configuration. However, the medial and/or lateral surfaces need not necessarily be substantially planar. For example, it can be preferable for the medial surface of the frame to be slightly convex. This can help to ensure that when implanted, the medial surface of the frame fits snugly against the concave configuration of the scapula in the glenoid region.

Preferably, the frame has a generally planar configuration and the bores are configured so that the distance between the axes of the bores, measured along each of two orthogonal measurement axes in the plane of the frame, decreases in a direction away from the medial surface of the frame, in which one of the measurement axes and the axis of the one of the bores lie in a common plane. This has been found to improve the anchorage of the frame within the scapula.

Preferably, the bores of the frame are configured so that the axes of the bores do not fall within a common plane. Preferably, the transverse distance measured between the axes decreases with increasing distance away from the medial surface of the frame towards the scapula.

Only one of the axes of the bores needs to be inclined relative to a first plane that extends perpendicular to the plane of the frame to ensure that the axes of the bores converge.

Preferably, the axis of the first bore and the axis of the second bore are both inclined relative to the first plane. This can be advantageous as it can improve the anchorage of the frame in the scapula.

Preferably, the axis of the first bore is inclined relative to the first plane by at least 5°, more preferably by at least 10°, especially preferably by at least 20°, most preferably by 24°. Preferably, the axis of the first bore is inclined relative to the first plane by not more than 35°, more preferably by not more than 30°, especially by not more than 25°.

Preferably, the axis of the second bore is inclined relative to the first plane by at least 10°, more preferably by at least 20°, especially preferably by at least 30°, most preferably by 33°. Preferably, the axis of the second bore is inclined relative to the first plane by not more than 50°, more preferably by not more than 40°, especially preferably by not more than 35°.

Preferably, the frame has a generally planar configuration and in which the bores are configured so that the distance between the axes of the bores, measured along each of two orthogonal measurement axes in the plane of the frame, decreases in a direction away from the medial surface of the frame, in which one of the measurement axes and the axis of one of the bores lie in a common plane. This has been found to improve the anchorage of the frame to the scapula.

Preferably, the axis of the first bore is inclined relative to first and second mutually perpendicular planes, the first and second planes being perpendicular to the plane of the frame when the frame is generally planar. Preferably, the axis of the second bore is inclined relative to the first and second mutually perpendicular planes, the first and second planes being perpendicular to the plane of the frame when the frame is generally planar. It has been found that this can improve the anchorage of the frame to the scapula. The minimum distance in both dimensions will be greater than 0 mm. The minimum distance in both dimensions will be sufficient for each screw to be received through the bores without colliding with other screws.

Preferably, the axis of the first bore is inclined relative to the second plane by at least 5°, more preferably by more than 7°, especially preferably by at least 10°, most preferably by 12°. Preferably, the axis of the first bore is inclined relative to the second plane by not more than 20°, more preferably by not more than 17°, especially preferably by not more than 15°.

Preferably, the axis of the second bore is inclined relative to the second plane by at least 1°, more preferably by at least 2°, most preferably by 3°. Preferably the axis of the second bore is inclined relative to the second plane by not more than 10°, more preferably by not more than 5°, especially preferably by not more than 4°.

Preferably, the angle between (a) a first line extending between the centre point of the first bore and the centre point of the frame, and (b) a second line extending between the centre point of the second bore and the centre point of the frame, is less than 180°. Such configuration of the bores can improve the anchorage of the frame to the scapula. Preferably, the angle between (a) and (b) is not more than 155°, especially preferably not more than 150°, most preferably 145°. Preferably, the angle between (a) and (b) is at least 100°, more preferably at least 120°, especially preferably at least 130°, most preferably at least 140°.

Preferably, the distance between the centre point of the first bore and the centre point of the frame, is at least 5 mm, more preferably at least 6 mm, especially preferably at least 7 mm, most preferably approximately 8 mm. Preferably, the distance between the centre point of the first bore and the centre point of the frame is not more than 11 mm, more preferably not more than 10 mm, especially preferably not more 9 mm.

Preferably, the distance between the centre point of the first bore and the centre point of the frame, is the same as the distance between the centre point of the second bore and the centre point of the frame.

Preferably, the bores of the frame are countersunk so that the heads of screws received therethrough do not protrude above the lateral surface. This can help prevent the heads of the screws interfering with the body part when the body is received on the frame.

The countersunk portion of the bores can be configured such that the head of a screw received through the bore can only be properly seated in the countersunk portion in one angular orientation. For example, the cross-sectional shape of the countersunk portion, in a plane perpendicular to the diameter of the bore can be square in shape. The countersunk portion can be configured such that when the head of the screw is properly seated in the countersunk portion, the axis of the screw is coaxial with the axis of the bore.

Preferably, the cross-sectional shape of the countersunk portion, in a plane perpendicular to the diameter of the bore, is such that a screw having a correspondingly shaped head can be properly seated in the bore in more than one angular orientation. The bores and the screws will be configured such that the screws can only be properly received in the frame when the screws are converged. Preferably, the cross-sectional shape is rounded so that the screw can be properly seated through a range of angles. This can be advantageous as it can allow for slight variations in the degree by which the screws converge and can give the surgeon some degree of freedom in choosing the location of the screws in the scapula. This can remove the need to have different shaped and sized frame parts for different patients having different shaped and sized scapulas.

Preferably, the axis of a screw received through a bore can not be inclined by more than 10° relative to the axis of the bore, more preferably not by more than 5°.

The diameter of the bores are sufficiently large so as to receive bone screws therethrough. The diameter of the bores can be configured such that the bone screws received therethrough can only be received in one angular orientation. Preferably, the diameter of the bores are slightly larger than the diameter of the shaft of the bone screws so that the bone screws can be received therethrough in a range of angular orientations.

The frame will generally be made from metallic based materials which are conventionally used in the manufacture of surgical implants. Certain stainless steels can be particularly preferred. Other materials include titanium and titanium alloys.

The implant components discussed above can be used in a method of shoulder joint replacement which comprises fastening a frame part of a glenoid component for a shoulder joint prosthesis to the scapula in the glenoid region thereof using bone screws which extend through the frame part into the lateral border pillar and the spine pillar of the scapula.

The method can include surgical planning steps, in which images are generated of the patient's glenoid to allow areas of high bone density to be identified. The information generated in from the images can enable the surgeon to position the screws visually with reference to visible landmarks. The images can also be used in a computer assisted surgical procedure, in which the positions of instruments used in the procedure are tracked relative to stored image data. The positions and orientations of the fixation screws can be identified relative to the bone tissue using the image data. The image data can be generated using techniques such as x-ray, computer tomography and magnetic resonance.

Embodiments of the present invention will now be described by way of example only with reference to the accompanying drawings in which:
Figure 1 is a schematic side view of a glenoid component for the right hand side of the body, comprising a frame part according to the present invention, fastened to the glenoid region of a scapula;
Figure 2 is a schematic perspective view of the frame part shown in Figure 1, from a first angular orientation;
Figure 3 is a schematic perspective view of the frame part shown in Figure 1, from a second angular orientation;
Figure 4 is a schematic side view of the frame part shown in Figure 1;
Figure 5 is a schematic plan view of the frame part shown in Figure 1; and
Figure 6 is a schematic side view of the frame part of the glenoid component shown in Figure 1, fastened to the glenoid region of the scapula.

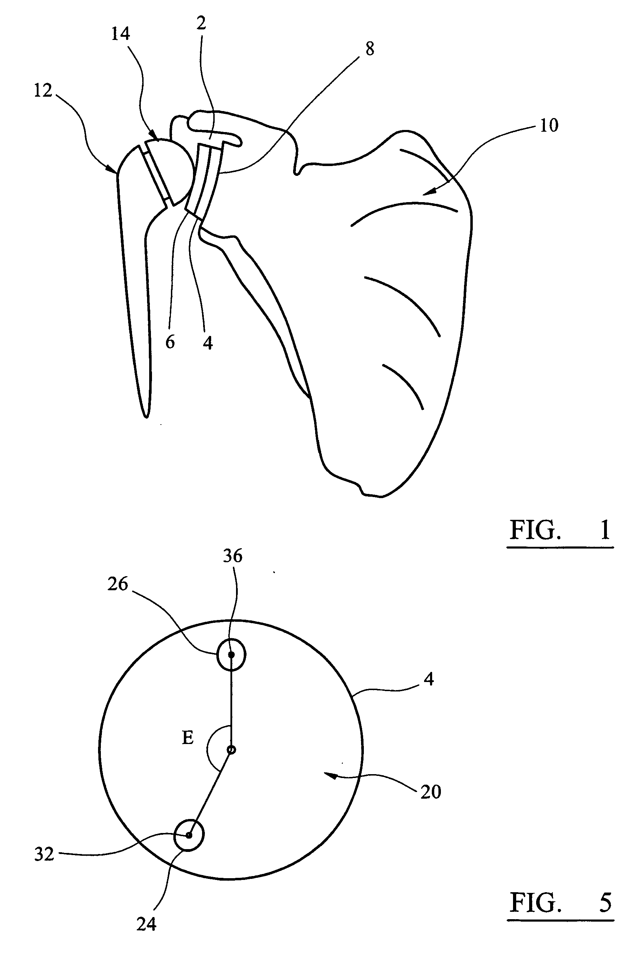

Referring to the drawings, Figure 1 shows a glenoid component 2 of a shoulder joint prosthesis, implanted in the glenoid region 8 of a scapula 10. Also shown is a humeral component 12 of the shoulder joint prosthesis, implanted in a humerus (not shown). The head 14 of the humeral component 12 is configured to articulate with the glenoid component 2.

The glenoid component 2, comprises a frame part 4 according to the present invention, and a body part 6. The body part 6 is fastened to the frame part 4, which in turn is secured to the glenoid region 8 of the scapula 10 by bone screws 16, 18 (not shown in figure 1). The head 14 of the humeral implant articulates with the surface of the body part 6 that is lateral relative to the scapula (i.e. the "lateral surface") when the implant 2 is implanted. The lateral surface of the body part is slightly concave.

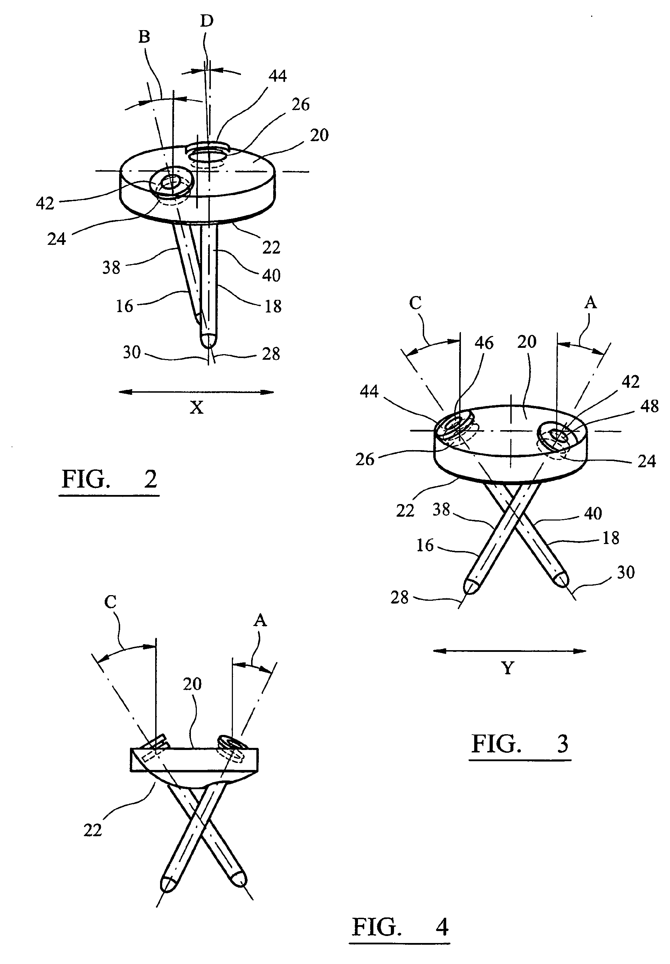

Figures 2 to 4 show the frame part 4 in more detail. The frame part 4 comprises a lateral surface 20 relative to the scapula 10 when the frame is implanted, (i.e. a "lateral surface"), and a medial surface 22 relative to the scapula 10 when the frame is implanted (i.e. a "medial surface"). As best shown in figure 4, the lateral surface 20 is planar, and the medial surface 22 is convex and rounded so as to match the anatomical configuration of the glenoid region 8. The frame has a generally planar configuration, the plane of the frame being parallel to the frame of the lateral surface 20.

The frame 4 further comprises first 24 and second 26 bores that extend between the lateral and medial surfaces for receiving the first 16 and second 18 bone screws. The bores are configured so that their axes 28, 30 are converged. As best shown in figures 2 and 3, the distance between the axes 28, 30, measured along each of two orthogonal measurement axes X and Y, in the plane of the frame, decreases as in a direction away from the medial surface 22 of the frame 4, and reaches a minimum distance along both measurement axes before diverging. The point along axes at which the distance between them is at a minimum is different along the X measurement axis than it is along the Y measurement axes.

As shown, the first bore 24 is configured so that its axis 28 is inclined relative to first and second mutually perpendicular planes, the first and second planes being perpendicular to the plane of the frame. As shown in Figure 4, the axis of the first bore 24 is inclined relative to the first plane by 24° (shown as angle A in Figure 4). The axis of the first bore 24 is inclined relative to the second plane by 12° (shown as angle B in Figure 3).

The second bore 26 is also configured so that its axis 30 is inclined relative to the same first and second mutually perpendicular planes. As shown in Figure 4, the axis of the second bore 26 is inclined relative to the first plane by 33° (shown as angle C in Figure 4). The axis of the second bore 26 is inclined relative to the second plane by 3° (shown as angle D in Figure 3).

As shown in Figure 5, the first 24 and second 26 bores are arranged within the frame such that the angle E between a first line extending between the centre point 32 of the first bore 24 and the centre point 34 of the frame 4, and a second line extending between the centre point 36 of the second bore 26 and the centre point of the frame, is 145°.

In the embodiment shown, the bores 24, 26 are configured so that the shank portions 38, 40 of the screws 16, 18 can be slidingly received therethrough. The diameter of the head portions 42, 44 of the screws are larger than the diameters of the bores, and therefore cannot be slide through the bores. The head portions 42, 44 of the screws have non-circular recesses 46, 48 formed therein for receiving a tool (not shown) for imparting a rotational force on the screws. In the embodiment shown, the bores 24, 26 are only partially countersunk. Therefore, a portion of the head portions 42, 44 of the screws protrude from the lateral surface 20 of the frame 4.

Due to the screws received through the bores of a frame according to the present invention being angularly inclined relative to the plane of the frame, it can be necessary in some circumstances to use screws longer than 18 mm to achieve sufficient anchorage within the scapula. Typically, the length of the bone screws 16, 18 will be at least about 18 mm, preferably at least about 24 mm, for example at least about 30 mm. The length will often be not more than about 120 mm, preferably not more than about 80 mm, more preferably not more than about 50 mm.

In use, the glenoid region 8 of the scapula 10 is prepared before implanting the glenoid implant 2. This includes preparing holes suitable for receiving the bone screws 16, 18 of the implant 2. Such holes can be prepared by using a suitable drilling tool and drilling guide. In some embodiments, the frame part 4 can be configured to enable the drilling guide to be fastened to the frame part 4, so that the drill guide and a bore of the frame are coaxial. Accordingly, in this way, the bore is also used as a drill guide. This can help ensure that the axis of the drilled hole in the bone is coaxial with the axis of the bore of the frame through which a screw will be received. However, it is also envisaged that the locations and orientations of the bone screw holes will be capable of being identified by the surgeon with reference to pre-operative images of the patient's anatomy and to landmarks on the anatomy which are identified in the images.

Once the glenoid region 8 and the holes for the bone screws 16, 18 are prepared, the frame 4 is secured to the scapula. The frame 4 is located on the glenoid region and the screws 16, 18 are passed through the frame 4 and screwed into the holes in the scapula. Once securely anchored, the body part 6 can be fastened to the frame part. Design features which can be used to fasten the body part to the frame part are known from existing orthopaedic prostheses which are constructed from separate modular parts, and include for example threaded fasteners, cooperating taper surfaces which fit together with an interference fit ("Morse taper"), etc.

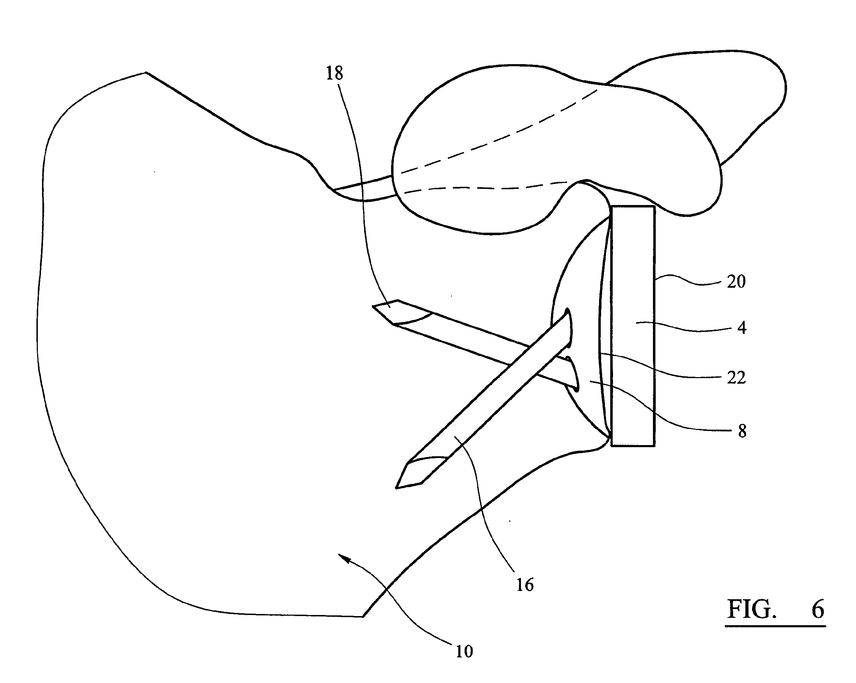

Figure 6 illustrates the frame part 4 secured to the scapula 10 by the first 16 and second 18 screws which extend into the lateral border pillar and the spine pillar of the scapula respectively.

## Claims

1. A method of planning a shoulder joint replacement surgical procedure which comprises:
a. obtaining scan image data of the patient's scapula,
b. identifying paths for fixation screws for a component of a shoulder joint prosthesis,
**characterised in that** the paths for the fixation screws extend into the lateral border pillar and the spine pillar of the scapula.

2. A method as claimed in claim 1, in which the paths are configured so that their axes converge, so that the distance measured between the axes decreases into the patient's scapula.

3. A method as claimed in claim 2, in which the axes of the bores do not fall within a common plane.

4. A method as claimed in claim 1, which includes the step of selecting a glenoid component (2) of a shoulder joint prosthesis for fixation to the glenoid region of a scapula, the component comprising a body part (6) of the prosthesis which presents a surface for articulation with the corresponding articulating surface of a humeral component, and a frame part (4) which has:
a lateral surface (20) on which the body part can be mounted;
a medial surface (22) generally opposite the lateral surface; and
firs (24) and second (26) bores that extend between the lateral and medial surfaces for receiving screws (16, 18) so as to fasten the frame part to the scapula;
in which the bores are configured so that their axes converge, so that the distance measured between the axes decreases with increasing distance away from the medial surface of the frame towards the scapula.

5. A method as claimed in claim 4, in which the frame has a generally circular configuration.

6. A method as claimed in claim 5, in which the bores are located inside the circular boundary of the frame.

7. A method as claimed in claim 1, in which the frame has a generally planar configuration, and in which the axis of the first bore is inclined relative to first and second mutually perpendicular planes, the first and second planes being perpendicular to the plane of the frame.

8. A method as claimed in claim 7, in which the axis of the second bore is inclined relative to the said first and second planes.

9. A method as claimed in claim 7, in which the angle between the axis of the first bore and the first plane is at least about 5°.

10. A method as claimed in claim 7, in which the angle between the axis of the first bore and the first plane is not more than about 35°.

11. A method as claimed in claim 7, in which the angle between the axis of the first bore and the second plane is at least about 5°.

12. A method as claimed in claim 7, in which the angle between the axis of the first bore and the second plane is not more than about 20°.

13. A method as claimed in claim 8, in which the angle between the axis of the second bore and the first plane is at least 10°.

14. A method as claimed in claim 8, in which the angle between the axis of the second bore and the first plane is not more than 50°.

15. A method as claimed in claim 8, in which the angle between the axis of the second bore and the second plane is at least 1°.

## Patentansprüche

1. Verfahren zum Planen einer chirurgischen Prozedur für das Ersetzen eines Schultergelenkes, das aufweist:
a. Erhalten von Abtastbilddaten des Schulterblattes des Patienten,
b. Identifizieren von Wegen für Fixierschrauben für eine Komponente einer Schultergelenkprothese,
**dadurch gekennzeichnet, dass** sich die Wege für die Fixierschrauben in die Außenrandgräte und die Schultergräte des Schulterblattes erstrecken.

2. Verfahren nach Anspruch 1, bei dem die Wege so ausgelegt sind, dass ihre Achsen zusammenlaufen, so dass sich der Abstand, der zwischen den Achsen gemessen wird, sich in das Schulterblatt des Patienten hinein verringert.

3. Verfahren nach Anspruch 2, bei dem die Achsen der Bohrungen nicht in eine gemeinsame Ebene fallen.

4. Verfahren nach Anspruch 1, welches den Schritt des Auswählens einer Glenoidkomponente (2) einer Schultergelenkprothese zum Fixieren an dem Glenoidbereich eines Schulterblattes aufweist, wobei die Komponente einen Körperteil (6) der Prothese, der eine Fläche für das Gelenkbilden mit der entsprechenden Gelenk bildenden Fläche einer Oberarmkomponente darstellt, und einen Rahmenteil (4) aufweist, der umfasst:
eine seitlich liegende Fläche (20), an der der Körperteil angebracht werden kann;
eine mittig liegende Fläche (22), im Allgemeinen der seitlich liegenden Fläche gegenüber angeordnet; und
eine erste (24) und eine zweite (26) Bohrung, die sich zwischen der seitlich und der mittig liegenden Fläche zum Aufnehmen von Schrauben (16, 18) erstrecken, um so den Rahmenteil an dem Schulterblatt festzulegen;
wobei die Bohrungen so ausgelegt sind, dass ihre Achsen zusammenlaufen, so dass der Abstand, der zwischen den Achsen gemessen wird, mit zunehmender Entfernung weg von der mittig liegenden Fläche des Rahmens auf das Schulterblatt zu abnimmt.

5. Verfahren nach Anspruch 4, bei dem der Rahmen eine im Allgemeinen kreisförmige Ausgestaltung hat.

6. Verfahren nach Anspruch 5, bei dem sich die Bohrungen innerhalb der kreisförmigen Begrenzung des Rahmens befinden.

7. Verfahren nach Anspruch 1, bei dem der Rahmen eine im Allgemeinen ebene Ausgestaltung hat und bei dem die Achse der ersten Bohrung in Bezug auf eine erste und eine zweite Ebene, die wechselseitig senkrecht zueinander sind, geneigt ist, wobei die erste und die zweite Ebene zur Ebene des Rahmens senkrecht stehen.

8. Verfahren nach Anspruch 7, bei dem die Achse der zweiten Bohrung in Bezug auf die erste und die zweite Ebene geneigt ist.

9. Verfahren nach Anspruch 7, bei dem der Winkel zwischen der Achse der ersten Bohrung und der ersten Ebene wenigstens ungefähr 5° beträgt.

10. Verfahren nach Anspruch 7, bei dem der Winkel zwischen der Achse der ersten Bohrung und der ersten Ebene nicht mehr als ungefähr 35° beträgt.

11. Verfahren nach Anspruch 7, bei dem der Winkel zwischen der Achse der ersten Bohrung und der zweiten Ebene wenigstens ungefähr 5° beträgt.

12. Verfahren nach Anspruch 7, bei dem der Winkel zwischen der Achse der ersten Bohrung und der zweiten Ebene nicht mehr als ungefähr 5° beträgt.

13. Verfahren nach Anspruch 8, bei dem der Winkel zwischen der Achse der zweiten Bohrung und der ersten Ebene wenigstens 10° beträgt.

14. Verfahren nach Anspruch 8, bei dem der Winkel zwischen der Achse der zweiten Bohrung und der ersten Ebene nicht mehr als 50° beträgt.

15. Verfahren nach Anspruch 8, bei dem der Winkel zwischen der Achse der zweiten Bohrung und der zweiten Ebene wenigstens 1° beträgt.

## Revendications

1. Procédé pour organiser une intervention chirurgicale de remplacement de l'articulation de l'épaule qui comprend les étapes consistant à :
a. obtenir des données d'image de balayage de l'omoplate du patient,
b. identifier les trajectoires pour les vis de fixation pour un composant d'une prothèse d'articulation de l'épaule,
**caractérisé en ce que** les trajectoires pour les vis de fixation s'étendent dans le pilier de bord latéral et le pilier de l'épine de l'omoplate.

2. Procédé selon la revendication 1, dans lequel les trajectoires sont configurées de sorte que leurs axes convergent, de sorte que la distance mesurée entre les axes diminue dans l'omoplate du patient.

3. Procédé selon la revendication 2, dans lequel les axes des alésages ne tombent pas à l'intérieur d'un plan commun.

4. Procédé selon la revendication 1, qui comprend l'étape consistant à sélectionner un composant glénoïde (2) d'une prothèse d'articulation de l'épaule pour la fixation à la région glénoïde d'une omoplate, le composant comprenant une partie de corps (6) de la prothèse qui présente une surface pour l'articulation avec la surface d'articulation correspondante d'un composant huméral, et une partie de châssis (4) qui a :
une surface latérale (20) sur laquelle la partie de corps peut être montée ;
une surface médiale (22) généralement opposée à la surface latérale ; et
des premier (24) et deuxième (26) alésages qui s'étendent entre les surfaces latérale et médiale pour recevoir les vis (16, 18) afin de fixer la partie de châssis à l'omoplate ;
dans lequel les alésages sont configurés de sorte que leurs axes convergent, de sorte que la distance mesurée entre les axes diminue en augmentant la distance de la surface médiale du châssis vers l'omoplate.

5. Procédé selon la revendication 4, dans lequel le châssis a une configuration généralement circulaire.

6. Procédé selon la revendication 5, dans lequel les alésages sont positionnés à l'intérieur de la limite circulaire du châssis.

7. Procédé selon la revendication 1, dans lequel le châssis a une configuration généralement plane, et dans lequel l'axe du premier alésage est incliné par rapport aux premier et deuxième plans mutuellement perpendiculaires, les premier et deuxième plans étant perpendiculaires au plan du châssis.

8. Procédé selon la revendication 7, dans lequel l'axe du deuxième alésage est incliné par rapport auxdits premier et deuxième plans.

9. Procédé selon la revendication 7, dans lequel l'angle entre l'axe du premier alésage et le premier plan est d'au moins environ 5°.

10. Procédé selon la revendication 7, dans lequel l'angle entre l'axe du premier alésage et le premier plan n'est pas supérieur à environ 35°.

11. Procédé selon la revendication 7, dans lequel l'angle entre l'axe et le premier alésage et le deuxième plan est au moins d'environ 5°.

12. Procédé selon la revendication 7, dans lequel l'angle entre l'axe du premier alésage et le deuxième plan n'est pas supérieur à environ 20°.

13. Procédé selon la revendication 8, dans lequel l'angle entre l'axe du deuxième alésage et le premier plan est d'au moins 10°.

14. Procédé selon la revendication 8, dans lequel l'angle entre l'axe du deuxième alésage et le premier plan n'est pas supérieur à 50°.

15. Procédé selon la revendication 8, dans lequel l'angle entre l'axe du deuxième alésage et le deuxième plan est d'au moins 1°.
